# EUROPEAN PATENT APPLICATION

(11) **EP 2 005 923 A1**
(43) Date of publication of application: **24.12.2008**
(21) Application number: 08158360.1
(22) Date of filing: 16.06.2008
(51) Int. Cl.: A61F 9/007, A61B 19/00, A61B 3/00

(54) **Apparatus, system and method for illuminated membrane manipulator**

(30) Priority: 19.06.2007 US 765036
(71) Applicant: Alcon, Inc., 6331 Hünenberg (CH)
(72) Inventor: Hickingbotham, Dyson W., Stouchsburg, PA 19567 (US)
(74) Representative: Hanna, Peter William Derek

(57) **Abstract**

Multifunctional surgical devices for use in ophthalmic surgery are disclosed. More particularly, embodiments of a surgical instrument with a multifunction tip are provided. Specifically, the tip may provide a source of illumination, a pik, and another tool such as a scraper, knife or spatula. This tool may be extended from the tip where the rigidity of the extendable tool varies with the distance it is extended from the tip.

## Description

### TECHNICAL FIELD OF THE INVENTION

The present invention relates to surgical devices. More particularly, embodiments of the present invention relate to multifunctional surgical devices for use in ophthalmic surgery. Even more particularly, embodiments of the present invention relate to illuminated surgical devices for the removal or manipulation of membranes during vitreoretinal or other ophthalmic surgical procedures.

### BACKGROUND

The human eye can suffer a number of maladies causing mild deterioration to complete loss of vision. While contact lenses and eyeglasses can compensate for some ailments, ophthalmic surgery is required for others. Generally, ophthalmic surgery is classified into posterior segment procedures, such as vitreoretinal surgery, and anterior segment procedures, such as cataract surgery. More recently, combined anterior and posterior segment procedures have been developed.

It is a frequent postoperative complication following surgical procedures in the posterior region of the eye that a glia cell membrane will form and attach itself to the retina. Anytime the choroid layer is stimulated such as by cutting, laser burning, lesion caused by probing or cryo or thermal damage, or any other form of stimulation, fibro and glia cells will be pumped from the choroid by pneumatic processes. Glia cells are glue-like and serve to repair damage surrounding the lesion formed by the stimulation. This excretion of glia cells is often used to "glue" a detached retina to the choroid. Unfortunately, the body's control mechanisms of secretion of fibro and glia cells are not finely tuned enough to stop secretion of the cells at the precise time when enough cells have been excreted to do the job. Excretion of excess cells often results in formation of a membrane of cells over the retina between the retina and the lens of the eye, ,resulting in obstruction of the light path to the retina. Sometimes, in the case of detached retinas, the membrane forms between the retina and the choroid.

The formation of these membranes is called proliferative disease. This disease frequently is a postoperative complication of surgical procedures for reattachment of detached retinas. Proliferative vitreoretinal disorders are usually manifested in the intraocular cavity of the eye by the growth of proliferative membranes on the neurosensory retina. More specifically, glia cells initially form a single-cell-layer membrane over the retina or between the retina and the choroid. This membrane is attached to the retina at various points by what are sometimes referred to as "nails". The retina is normally attached to the choroid by a structure which is similar to VELCRO.TM. brand hook-and-loop fabric.

There is a problem of spontaneous detachment of the retina if proliferative disease develops. This can occur for the following reason. The membranes initially form as a single cell layer. Later, the cells often rearrange themselves into a multiple cell layer without growing further. Attachments to the retina at the locations of the "nails" may be quite strong. Because the same number of cells that were formerly a single cell layer have become a layer of multiple cell thickness, the membrane tends to shrink. This shrinkage causes stresses on the retina at the positions of the "nails" which can result in holes being pulled in the retina or in spontaneous detachment of the retina from the choroid.

To address these issues surgeons usually remove these membranes utilizing a variety of techniques. For example, one such technique is to take a vertical intraocular scissors with the blades closed and insert the tip of the scissors between the membrane and the retina. After the scissors are inserted, the blades are very gently opened to separate the membrane from the retina. Another technique is to utilize an instrument with a hook to separate the membrane from the retina by pulling gently on the membrane. Still another technique is to uses an abrasive media on a tip of the microsurgical instrument to scrape the membranes from the surface of the retina.

The difficulty with these prior art approaches is that damage to the retina can easily result. One type of damage can result if the mechanical forces applied to the membrane and transmitted to the retina at the locations of the "nail" are too large. This can result in holes being pulled in the retina at the position of the "nails". Further, detachment of the retina from the choroid can also result if the force exerted on the retina through the nails exceeds the force holding the retina to the choroid. Another type of damage that can result is through inadvertent movement of the instrument while the retina is engaged on the hook, which results in excessive force being applied to the retina. If the tools are not manipulated with great skill, holes can be poked in the retina. These holes result from the relatively concentrated forces acting on the retina at the tips of the instruments. Even a slight misapplication of force can result in this type of damage to the retina.

However, the use of the aforementioned surgical techniques may require the use of multiple instruments while performing the surgery such as the types of instruments used in performing the surgery described above (scissors, hooks, etc.), instruments designed to deliver an aspiration source locally to an area to remove tissue, instruments designed to aid in the visualization of the surgical site, etc.

Thus, during the course of an operation, a surgeon must alternate between instruments to carry out the specific function required at a particular stage of the operation. Switching between such instruments is not only inconvenient and time consuming, but additionally may increase the likelihood of mistakes occurring and resulting retinal damage.

### SUMMARY OF THE INVENTION

Attention is now directed to embodiments of the present invention, which are directed to multifunctional surgical devices for use in ophthalmic surgery. More particularly, embodiments of the present invention may provide a surgical instrument with a multifunction tip. Specifically, the tip may provide a source of illumination such as a portion of a fiber optic cable and a portion of the tip may be shaped as a pik. Furthermore, the instrument may be operated to extend another tool from the tip such as a scraper, knife or spatula where the rigidity of the extendable tool varies with the distance it is extended from the tip. Thus, by providing multiple functionality in a single surgical instrument (e.g. illumination, a pik, and scraping functionality) a variety of tasks may be accomplished utilizing a single surgical instrument, reducing changeovers between surgical instruments during a procedure which, in turn, may simplify the procedure and reduce both the likelihood of complications and the harmful stresses placed on the eye during the surgery.

In one embodiment, a surgical instrument may comprise a handle and a tip coupled to the handle where an opening is formed in the tip. A portion of a fiber optic cable may protrude from the opening. Furthermore, the end of the opening distal is shaped as a pik and the tip comprises a tool such as a scraper, knife, or spatula, where the tool is extendable to varying lengths beyond the tip.

During use of such a surgical instrument the protruding portion of the fiber optic cable may provide illumination to a surgical site while a surgeon may perform the surgical procedure utilizing the pik and the tool provided by the tip of the surgical instrument. By utilizing a multifunction surgical tool a surgeon may more effectively manipulate a membrane, allowing the surgeon to better control membrane removal or manipulation without the need to change surgical instruments.

These and other aspects of the invention will be better appreciated and understood when considered in conjunction with the following description and the accompanying drawings. The following description, while indicating various embodiments of the invention and numerous specific details thereof, is given by way of illustration and not of limitation. Many substitutions, modifications, additions, or rearrangements may be made within the scope of the invention, and the invention includes all such substitutions, modifications, additions, or rearrangements.

### BRIEF DESCRIPTION OF THE FIGURES

A more complete understanding of the present invention and the advantages thereof may be acquired by referring to the following description, taken in conjunction with the accompanying drawings in which like reference numbers indicate like features and wherein:
FIGURE 1 is a diagrammatic representation of one embodiment of a surgical console;
FIGURE 2 is a representation of one embodiment of a surgical instrument;
FIGURE 3 is a representation of one embodiment of a tip of a surgical instrument;
FIGURE 4 is a representation of one embodiment of a tip of a surgical instrument;
FIGURE 5 is a representation of one embodiment of a tip of a surgical instrument;
FIGURE 6 is a representation of one embodiment of a tip of a surgical instrument; and
FIGURE 7 is a representation of one embodiment of a tip of a surgical instrument.

### DETAILED DESCRIPTION

Preferred embodiments of the invention are illustrated in the FIGURES, like numerals being used to refer to like and corresponding parts of the various drawings.

Attention is now directed to embodiments of the present invention, which are directed to multifunctional surgical devices for use in ophthalmic surgery. More particularly, embodiments of the present invention may provide a surgical instrument with a multifunction tip. Specifically, the tip may provide a source of illumination such as a portion of a fiber optic cable and a portion of the tip may be shaped as a pik. Furthermore, the instrument may be operated to extend another tool from the tip such as a scraper, knife or spatula where the rigidity of the extendable tool varies with the distance it is extended from the tip. Thus, by providing multiple functionality in a single surgical instrument (e.g. illumination, a pik, and scraping functionality) a variety of tasks may be accomplished utilizing a single surgical instrument, reducing changeovers between surgical instruments during a procedure which, in turn, may simplify the procedure and reduce both the likelihood of complications and the harmful stresses placed on the eye during the surgery.

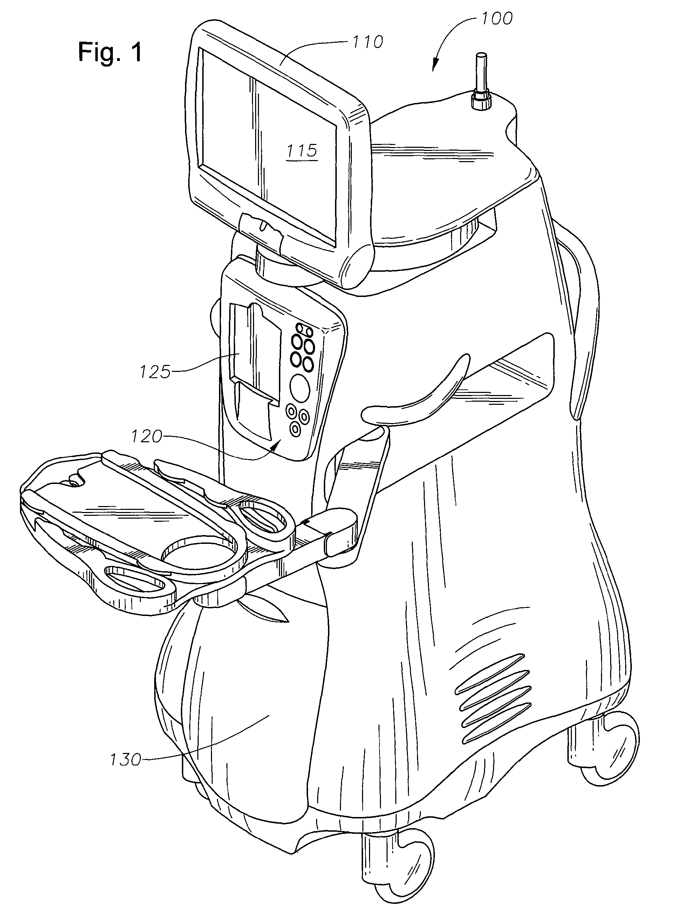

In one embodiment, an embodiment of a surgical instrument in accordance with the present invention may be utilized in conjunction with an ophthalmic surgical console. FIGURE 1 is a diagrammatic representation of one embodiment of just such an ophthalmic surgical console 100. Surgical console 100 can include a swivel monitor 110 that has touch screen 115. Swivel monitor 110 can be positioned in a variety of orientations for whoever needs to see touch screen 115. Swivel monitor 110 can swing from side to side, as well as rotate and tilt. Touch screen 115 provides a GUI that allows a user to interact with console 100.

Surgical console 100 also includes a connection panel 120 used to connect various tools and consumables to surgical console 100. Connection panel 120 can include, for example, a coagulation connector, balanced salt solution receiver, connectors for various handpieces, and a fluid management system ("FMS") or cassette receiver 125. Surgical console 100 can also include a variety of user- friendly features, such as a foot pedal control (e.g., stored behind panel 130) and other features.

Surgical console 100 is provided by way of example and embodiments of the present invention can be implemented with a variety of surgical systems or as stand-alone devices. Example surgical systems with which various embodiments of the present invention can be used include, for example, the Series 2000® Legacy® cataract surgical system, the Accurus® 400VS surgical system, and the Infiniti™ Vision System surgical system, all available from Alcon Laboratories Inc. of Fort Worth, Texas.

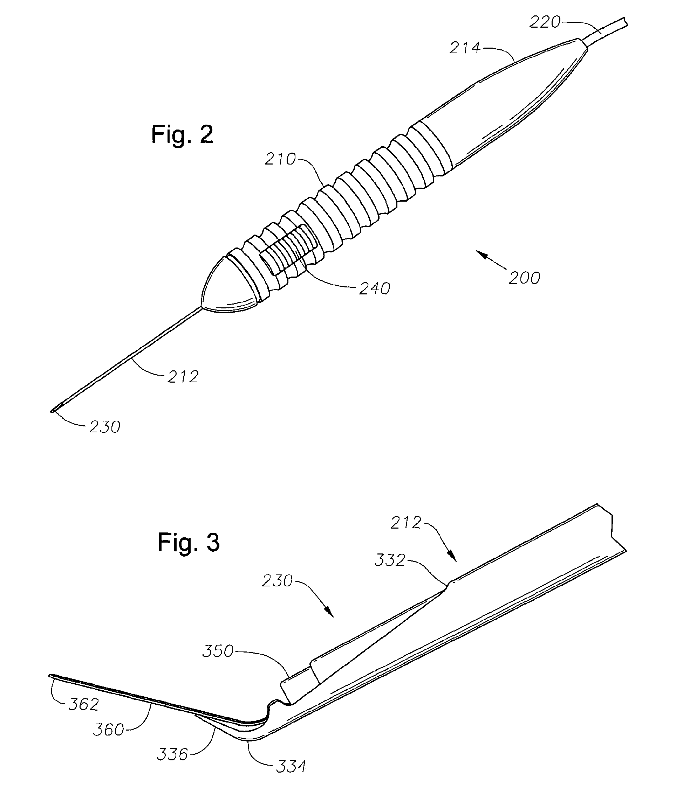

Turning now to FIGURE 2, one embodiment of a surgical instrument with a multifunction tip is depicted. Surgical instrument 200 comprises handle 210 and tip 212 coupled to handle according to any means known in the art such as a press-on fitting, screw-on fitting, etc. or alternatively tip 212 may be integrally formed with handle 210. Tip 212 comprises a hollow tube, which may be on the order of 20 or 23 gauge stainless steel, nickel, plastic, or the like. The end 214 of the handle 210 distal to tip 212 is coupled to a fiber optic cable 220 (portions of which may be covered with a protective sheath as shown in FIGURE 2), the fiber optic cable comprising one or more glass, plastic, or other type of optic fibers. The end (not shown) of fiber optic cable 220 distal from handle 210 may be coupled to a light source (e.g. through connection panel 120) such that the fiber optic cable 220 may conduct the light from the light source, as is known in the art. Fiber optic cable 220 may be routed through a passage in handle 210 configured to accept the fiber optic cable 220 to the end of handle 210 proximal to tip 212 and through tip 212, such that an unsheathed portion of fiber optic cable protruding from opening 230 at the end of tip 212 distal from handle 210 may radiate the conducted light. Tip 212 may also comprise a tool such as a membrane scraper, knife, delamination spatula, or the like, which may be extended to varying lengths between a fully extended position beyond opening 230 or a fully retracted position in hollow tip 212 such that no portion of the tool extends beyond the end of the opening 230 in the fully retracted position. This tool may be operatively coupled to button 240 on handle 210, where the actuation of button 240 controls the extension or retraction of the tool from tip 212. The coupling between button 240 and the tool may, for example, be a direct mechanical coupling. The button may actuate a motor which may, in turn, extend or retract the tool, or another method of operatively coupling the button 240 to the tool may be utilized such as a trigger or squeeze mechanism. In one embodiment, the protruding portion of fiber optic cable may extend or retract in conjunction with the extension or retraction of the tool, such that the amount of fiber optic cable 220 which protrudes from opening 230 may vary directly with the extension length of the tool. Thus when the tool is fully extended the protruding portion of fiber optic cable 220 is fully extended and when the tool is fully retracted the protruding portion of fiber optic cable 220 may be fully retracted (though a portion of fiber optic cable 220 may still protrude from opening 230 in the fully retracted position). In embodiments such as these, fiber optic cable 220 may also be operatively coupled to button 240 on handle 210 where the actuation of button 240 controls the extension or retraction of the protruding portion of fiber optic cable 220 from opening 230.

Moving now to FIGURE 3, one embodiment of a tip for use with surgical tool 200 which comprises a membrane scraper is depicted. Tip 212 comprises opening 230 which begins at end 332 of opening proximal to handle 210 and end 334 distal from handle 210. Opening 230 may be an elliptical (e.g,. the shape of the opening may be defined by an ellipse forming an angle with the longitudinal access of tip 212) or canted (e.g. relative to the longitudinal axis of tip 212) opening and end 334 of opening 230 distal from handle 210 may be formed into pik 336, while portion 350 of fiber optic cable 220 may protrude from end 332 of opening 230 proximal to handle 210. Fiber optic cable 220 may, for example, comprise a 100 micron optic fiber where the portion 350 of fiber optic cable 220 protruding from opening 230 may be shaped to radiate conducted light in a desired manner, such that desired illumination of a surgical site may be obtained during use. Additionally, in one embodiment tip 212 may comprise membrane scraper 360, which may be extended varying lengths beyond pik 336 at end 334 of opening 230 or retracted into tip 212 (e.g., by actuation of button 240 on handle 210), where the stiffness or rigidity of membrane scraper 360 varies inversely with its extension length. In other words, the longer membrane scraper 360 extends beyond pik 336 the more pliable or flexible it may become. Notice here that membrane scraper 360 is in an extend position as is protruding portion 350 of fiber optic cable 220.

In one embodiment, membrane scraper 360 may, for example, be comprised of a .002 inch or 50µm polyimide sheet, silicon, or another type of flexible rubber or polymer. The material comprising membrane scraper 360 may be translucent or semi-translucent, such that membrane scraper 360 may itself conduct at least a portion of light radiated by protruding portion 350 of fiber optic cable 220. An abrasive surface for achieving desired membrane scraping, manipulation, or removal may also be formed, coated on, or otherwise bonded or adhered to, abrasive portion 362 of membrane scraper 360 in a manner where the abrasive material will not separate from membrane scraper 360 during use. The abrasive material may comprise diamond particles (e.g., synthetic diamonds or the like) on the last 5 to 3mm of membrane scraper 360 distal to handle 210. In other embodiments the abrasive surface may comprise an abrasive pattern formed on abrasive portion 362 of membrane scraper 360, where this abrasive pattern comprises perforated holes formed in the abrasive portion 362 of membrane scraper 360 or another suitable abrasive pattern such as a stamp of a diamond pattern surface.

During use of surgical instrument 200, fiber optic cable 220 may be connected to a light source, such that protruding portion 350 of fiber optic cable 220 radiates light conducted along fiber optic cable 220 from the light source. Additionally, portions of light radiated from protruding portion 350 of fiber optic cable 220 may be conducted by and radiated from membrane scraper 360. Thus, when utilizing surgical instrument 200 illumination may be provided to a surgical site by light radiated by protruding portion of 332 of fiber optic cable 220 or membrane scraper 360, such that a surgeon may more effectively manipulate a membrane using adjustable stiffness membrane scraper 360 or pik 336 allowing the surgeon to better control membrane removal or manipulation and the flexibility to either use adjustable stiffness membrane scraper 360 or pik 336 for the manipulation or removal without the need to change surgical instruments

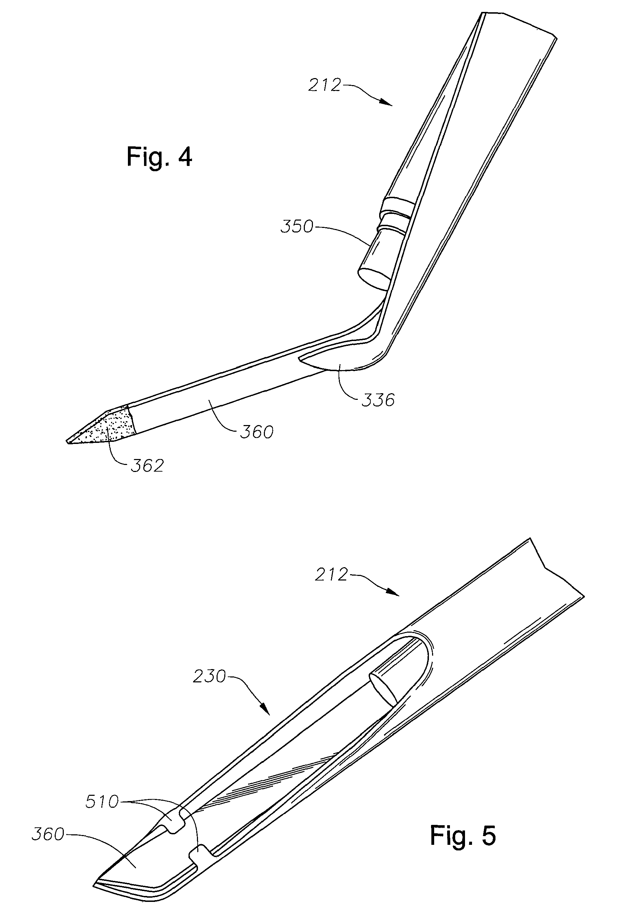

It may be helpful to an understanding of embodiments of the surgical instruments depicted herein to show different views of certain embodiments. To that end, FIGURE 4 depicts another view of an embodiment of a tip of a surgical instrument which comprises a membrane scraper. Notice with respect to the depicted view that pik 336 is formed at an angle with respect to longitudinal axis of tip 212. Notice also that, in one embodiment, membrane scraper 350 may extend from tip 212 at substantially the same angle as pik 336. As can be seen with respect to FIGURE 4, the end of protruding portion 350 of fiber optic cable 220 (which is here depicted in the extend position) may radiate conducted light into membrane scraper 360, such that membrane scraper 360 may conduct at least a portion of this radiated light to end of membrane scraper 360 distal from tip 212, providing better illumination to a surgical site when utilizing membrane scraper 360 in a surgical procedure, as discussed above.

FIGURE 5 depicts a close- up view of an opening of an embodiment of a tip of a surgical instrument comprising a membrane scraper where the membrane scraper is retracted into the tip and the protruding portion of the fiber optic cable is likewise in a retracted position. Notice with respect to FIGURE 5 that tip 212 may also have tabs 510 formed over opening 230. These tabs may keep membrane scraper 360 (or another tool) reinforced (i.e., properly positioned or supported), for example when membrane scraper 360 is in a partially or fully extended position.

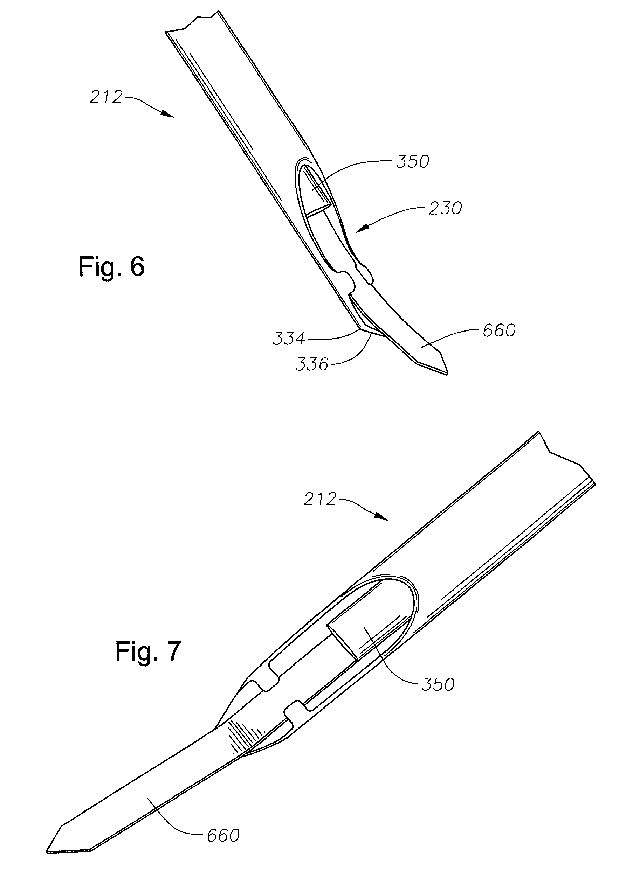

As mentioned above, embodiments of surgical instrument 200 may comprise a variety of tools in lieu of a membrane scraper. Embodiments such as these will be better understood with reference to FIGURE 6 which depicts one embodiment of a tip which comprises a knife. Here, tip 212 may be similar to other embodiments described above and comprise opening 230, end 334 having a pik 336 and a protruding portion 350 of fiber optic cable 220, such that desired illumination of a surgical site may be obtained during use. In the embodiment depicted, tip 212 may comprise knife 660, which may be extended varying lengths beyond end 334 of opening 230 or retracted into tip 212 where the stiffness or rigidity of knife 660 varies inversely with the length it is extended. Knife 660 may, for example, be comprised of .002 inch or 50µm thick stainless steel or the like.

In use, fiber optic cable 220 may be connected to a light source, such that protruding portion 350 of fiber optic cable 220 radiates light conducted along fiber optic cable 220 from the light source. Thus, when utilizing surgical instrument 200, illumination may be provided to a surgical site by light radiated by protruding portion of 350 of fiber optic cable 220 such that a surgeon may more effectively manipulate a membrane using knife 660 or pik 336 allowing the surgeon to better control membrane removal or manipulation and the flexibility to use either adjustable stiffness knife 660 or pick 336 for the manipulation or removal of a membrane without the need to change surgical instrument. FIGURE 7 depicts another view of an embodiment of a tip of a surgical instrument comprising a knife.

Although the present invention has been described in detail herein with reference to the illustrated embodiments, it should be understood that the description is by way of example only and is not to be construed in a limiting sense. It is to be further understood, therefore, that numerous changes in the details of the embodiment of this invention and additional embodiments of this invention will be apparent, and may be made by persons of ordinary skill in the art having reference to this description. It is contemplated that all such changes and additional embodiments are within scope of the invention as claimed below.

## Claims

1. A surgical instrument comprising:
a handle;
a tip coupled to the handle, comprising an opening and a tool disposed with the tip, wherein an end of the opening distal to the handle is shaped as a pik and the tool is extendable to varying lengths between a retracted position within the tip and an extended position; and
a fiber optic cable routed through the handle and the tip, wherein a portion of the fiber optic cable protrudes from the opening.

2. The surgical instrument of claim 1, wherein a stiffness of the tool varies with an extension length of the tool.

3. The surgical instrument of claim 2, wherein the portion of the fiber optic cable varies with the extension length of the tool.

4. The surgical instrument of claim 3, wherein the extension length of the tool and the portion of the fiber optic cable is controlled through the handle.

5. The surgical instrument of claim 2, further comprising one or more tabs, wherein the tabs reinforce the tool.

6. The surgical instrument of claim 2, wherein the tool is a membrane scraper.

7. The surgical instrument of claim 6, wherein an end of the membrane scraper distal from the handle comprises an abrasive surface.

8. The surgical instrument of claim 7, wherein the abrasive surface comprises a diamond coating.

9. The surgical instrument of claim 7, wherein the abrasive surface comprises an abrasive pattern.

10. The surgical instrument of claim 7, wherein the membrane scraper is a polyimide.

11. The surgical instrument of claim 10, wherein the membrane scraper is approximately 50µm thick.

12. The surgical instrument of claim 10, wherein the membrane is semi-translucent.

13. The surgical instrument of claim 2, wherein the tool is a knife.

14. The surgical instrument of claim 13, wherein the knife is approximately 50µm thick.

15. The surgical instrument of claim 2, wherein the tool is a delamination spatula.
